(19) Europäisches Patentamt

European Patent Office

Office européen des brevets

(11) Veröffentlichungsnummer: **0 413 187 B1**

(12) **EUROPÄISCHE PATENTSCHRIFT**

(45) Veröffentlichungstag der Patentschrift: **12.04.95**

(21) Anmeldenummer: **90114575.5**

(22) Anmeldetag: **30.07.90**

(51) Int. Cl.⁶: **A61K 39/395**

(54) Verfahren zur Herstellung nicht modifizierter intravenös verabreichbarer IgM- und/oder IgA-haltige Immunglobulinpräparate.

(30) Priorität: **17.08.89 DE 3927112**

(43) Veröffentlichungstag der Anmeldung:
**20.02.91 Patentblatt 91/08**

(45) Bekanntmachung des Hinweises auf die Patenterteilung:
**12.04.95 Patentblatt 95/15**

(84) Benannte Vertragsstaaten:
**AT BE CH DE ES FR GB IT LI NL SE**

(56) Entgegenhaltungen:
**EP-A- 0 013 901**
**EP-A- 0 352 500**
**US-A- 3 808 189**

**PATENT ABSTRACTS OF JAPAN, Band 9, Nr. 21 (C-263)[1744], 29. Januar 1985&NUM;**

**PATENT ABSTRACTS OF JAPAN, Band 13, Nr. 228 (P-877)(3576), 26. Mai 1989&NUM;**

(73) Patentinhaber: **BIOTEST PHARMA GMBH**
**Landsteiner Strasse 5**
**D-63303 Dreieich (DE)**

(72) Erfinder: **Kotitschke, Ronald, Dipl.-Chem.Dr.**
**Kleiststrasse 23**
**D-6072 Dreieich (DE)**
Erfinder: **Stephan, Wolfgang, Dipl.-Chem.Dr.**
**Philipp Holzmann-Strasse 84**
**D-6072 Dreieich (DE)**
Erfinder: **Möller, Wolfgang, Dipl.-Chem.Dr.**
**Graf von Stauffenberg-Strasse 32**
**D-6370 Oberursel (DE)**
Erfinder: **Piechaczek, Detlef, Dipl.-Chem.Dr.**
**Darmstädter Strasse 54**
**D-6115 Münster (DE)**
Erfinder: **Rudnick, Dieter, Dipl.-Chem.Dr.**
**Freiherr vom Stein-Strasse 1**
**D-6072 Dreieich (DE)**

(74) Vertreter: **Beil, Hans Chr., Dr. et al**
**BEIL, WOLFF & BEIL,**
**Rechtsanwälte,**
**Postfach 80 01 40**
**D-65901 Frankfurt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung einer für die intravenöse Applikation geeigneten Immunglobulinlösung durch Behandlung einer durch Fraktionierung aus Humanblut erhaltenen Proteinfraktion, die die Immunglobuline des Typs IgG, IgA und IgM in ankonzentrierter Form enthält.

In der EP 0 013 901 ist ein Verfahren zur Herstellung einer intravenös anwendbaren IgM-haltigen Proteinlösung beschrieben, bei dem eine durch Fraktionierung aus Blutplasma oder Serum erhaltene Proteinfraktion (wie z.B. eine Cohn-Fraktion III) durch Behandlung mit kolloidaler Kieselsäure von Lipiden befreit, mit Diethylaminoethyl-Gruppen tragenden, quervernetzten Dextranen oder Cellulose, vorzugsweise DEAE-Sephadex A 50, behandelt und dann mit ß-Propiolacton bei Temperaturen von 20 bis 37° C und pH-Werten von 7.0 bis 8.5 für die Dauer von 2 bis 10 Stunden bis zur pH-Konstanz behandelt wird. Die Cohn-Fraktion III enthält neben den gewünschten Immunglobulinen erhebliche Mengen von denaturiertem Protein, Lipide und viele Proteasen. Für die intravenöse Unverträglichkeit der Cohn-Fraktion III sind IgG-Polymere sowie die Proteasen mit hohen proteolytischen Aktivitäten und andere denaturierte Proteine verantwortlich. Die in EP 0 013 901 verwendete kolloidale Kieselsäure ist ein geeignetes Mittel zur Entfernung von Lipiden, führt jedoch zu Kontaktaktivierungen der Gerinnungsfaktoren und erhöht somit zusätzlich die in der Cohn-Fraktion III enthaltenen Mengen an proteolytisch wirksamen Proteinen. Die Adsorption dieser Proteasen macht daher im Verfahren der EP 0 013 901 die Verwendung eines Anionenaustauschers vom Typ DEAE-Sephadex A 50 erforderlich. Bei unspezifischen Aktivierungen von Proteasen ist eine sichere quantitative Entfernung dieser Proteasen häufig nur durch die Verwendung unverhältnismäßig großer Mengen des entsprechenden Adsorbens möglich. Es ist auch nicht auszuschließen, daß bei Kontaktaktivierungen der Proteine des Gerinnungssystems und des Kininsystems proteolytische Aktivitäten freigesetzt werden, die nicht an Anionenaustauscher binden.

Der vorliegenden Erfindung lag die Aufgabe zugrunde, ein Verfahren zur Herstellung einer sterilen und intravenös anwendbaren Lösung der Immunglobuline IgG, IgA und IgM unter Verwendung der Cohn-Fraktion III oder einer durch Fraktionierung aus Blutplasma oder Serum erhaltenen Proteinfraktion zu finden, die die Immunglobuline in angereicherter Form enthält.

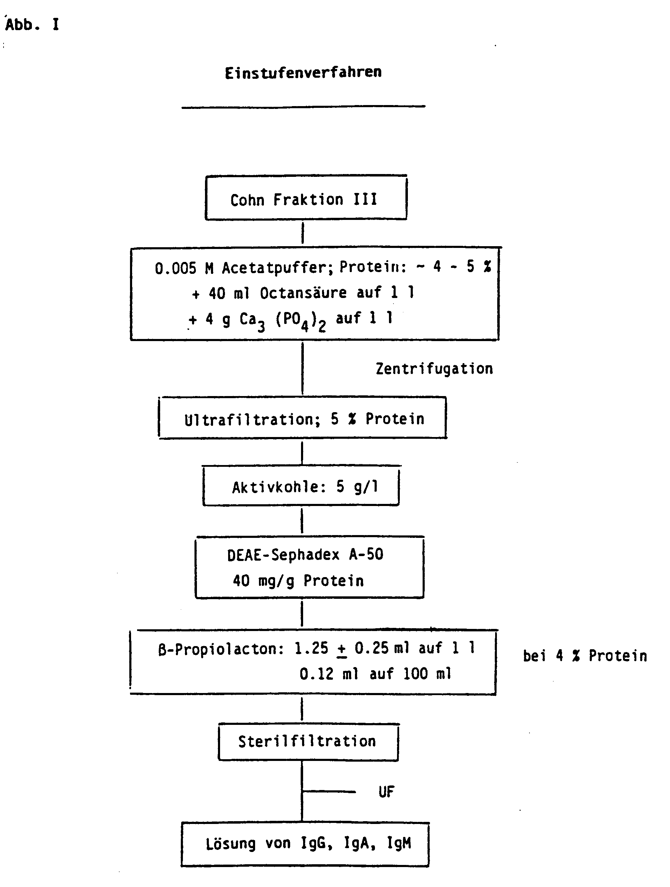

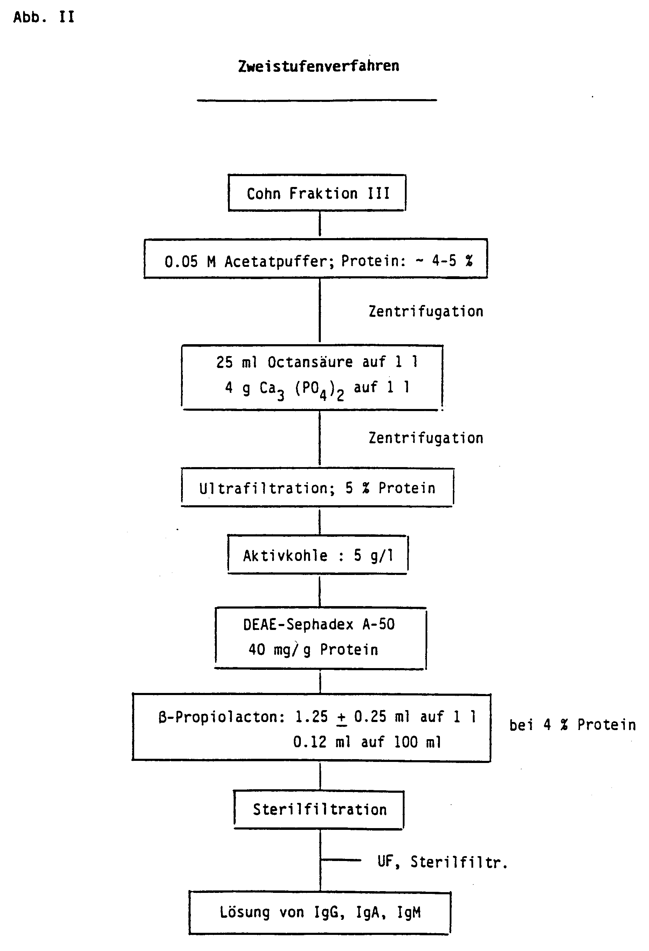

Überraschenderweise wurde nun gefunden, daß man zur Herstellung einer intravenös anwendbaren Lösung der Immunglobuline IgG, IgA und IgM auf die Entfernung der Lipide aus der Cohn-Fraktion III durch die Adsorption an kollodialer Kieselsäure verzichten kann. Die Abbildung I (Anhang) zeigt schematisch das erfindungsgemäße Verfahren, in Abbildung II (Anhang) ist eine Variante dargestellt.

Erfindungsgemäß wird dabei zunächst die Proteinfraktion mit Acetatpuffer versetzt, anschließend mit Calciumphosphat und Octansäure behandelt und zentrifugiert, der Überstand abgetrennt, mit Aktivkohle behandelt und sodann einer Adsorption an DEAE-Sephadex A50$^{(R)}$ unterworfen und die so erhaltene Lösung sterilfiltriert.

Die Aufarbeitung der Cohn-Fraktion III gemäß der EP 0 013 901 führt zu einer intravenös verträglichen Lösung, die die Immunglobuline in folgender Mengenverteilung enthält: IgG: 4000 mg %; IgA: 500 mg % und IgM: 500 mg %, bei einem Gesamtproteingehalt von 5 g %. Das Verhältnis von IgG zu IgM in diesem Präparat beträgt also 8 : 1. Als wirksamer Bestandteil dieses Produktes bei der Behandlung bakterieller Infektionen ist nicht nur das IgM-Molekül beteiligt, sondern auch das im Produkt enthaltene IgG.

Humanes IgG besteht aus vier Subklassen (IgG-1, IgG-2, IgG-3 und IgG-4) mit unterschiedlichen chemischen und biologischen Eigenschaften. Nach einem primären Kontakt mit Bakterien werden zuerst Antikörper vom IgM-Typ produziert, die die Bakterien zur Agglutination bringen und schließlich eine Komplement-abhängige Lysis bewirken. Die später auftretenden IgG-Antikörper, die normalerweise eine höhere Affinität zum Antigen besitzen, abhängig von der IgG-Subklasse, induzieren eine Fc-Rezeptor-abhängige Phygozytose oder Zerstörung via Komplement (F. Shakib, Derby: Basic und Clinical Aspects of IgG-Subclasses; in Monographes in Allergy, Vol. 19 (1986), Karger).

Die IgG-Subklassen-Verteilung nach bakteriellen Infektionen ist in einigen Studien untersucht worden, hauptsächlich jedoch nach Vaccinationen. Die Zuordnung bestimmter Antikörperaktivitäten zu bestimmten IgG-Subklassen ist heute noch ein Feld wissenschaftlicher Forschung. Solange die Zuordnung jedoch nicht möglich ist, ist es wünschenswert, in IgG-haltigen Immunglobulinpräparaten eine IgG-Subklassenverteilung zu erreichen, die möglichst derjenigen eines normalen Poolplasmas entspricht.

Die Tabelle I (Anhang) zeigt, daß die Verwendung von kolloidaler Kieselsäure während der Herstellung von IgG-haltigen Immunglobulinpräparaten zu einer Abreicherung bis hin zum Verlust der IgG-Subklasse 3 führt.

Die Bestimmung der Immunglobulin-Konzentration erfolgte mit folgenden verschiedenen Methoden: Die Mancini-Technik mit Partigenplatten $^{R}$) der Behringwerke und Quantiplate $^{R}$) von Kallestad und dem Nephelometer Auto ICS II von Beckmann. Referenzmaterial für IgG, IgA und IgM war der WHO-Standard 67/86, in dem 100 IU/ml enthalten waren. Die IgG-Subklassen wurden mit der radialen Immundiffusion auf Agarose-Platten, unter Verwendung polyclonaler Subklassen-Antiseren (Schaf) bestimmt (Fa. Janssen). Als Referenzserum diente der für die Immunglobulin-Bestimmung WHO-Referenzserumpool, da bis heute kein offizieller WHO-Standard für die IgG-Subklassen-Bestimmung vorhanden ist. Die Gesamtlipide wurden mit Reagenzien von der Fa. E. Merck bestimmt. Die Proteinlösung wurde mit konzentrierter Schwefelsäure erhitzt und anschließend mit Phosphorsäure-Vanillin-Reagenz umgesetzt.

Die Verwendung von kolloidaler Kieselsäure in einer Konzentration von 3% Aerosil gemäß der EP 0 013 901 führt zu der Abreicherung unerwünschter Lipide, bewirkt aber ebenfalls den Verlust der IgG-Subklasse 3.

Die Eigenschaft der kolloidalen Kieselsäure, die IgG-Subklasse 3 bevorzugt aus Proteinlösungen zu adsorbieren, betrifft nicht nur, wie in Tab. I aufgeführt, bereits hochgereinigte IgG-Lösungen, sondern trifft generell auf IgG-haltige Proteinlösungen zu (Tab. II, Anhang).

Das erfindungsgemäße Produkt enthält die IgG-Subklasse 3 in einer Konzentration, die einem Normalserumpool entspricht, während einem mit 3% Aerosil [R]) (AE)-behandelten Präparat die IgG-Subklasse 3 fehlt, wie in der folgenden Tab. III dargestellt ist.

Tab.III

| IgG-Subklassenverteilung in % | | | | |
|---|---|---|---|---|
| | IgG-1 | IgG-2 | IgG-3 | IgG-4 |
| IgG ohne AE-Behandlung | 58.8 | 28.1 | 3.7 | 9.4 |
| IgG mit AE-Behandlung | 61.2 | 29.5 | 0.1 | 9.2 |
| Normalserum IgG* | 70-60 | 25-27 | 2.4-4.6 | 1.6-7.5 |

* Geigy Tabelle (1979) 125

Blutplasmafraktionen haben ein hohes Risiko infektiös zu sein. Dieses Risiko betrifft besonders die Übertragung der Hepatitis B und Hepatitis Non-A/Non-B und in jüngster Zeit der HIV (human immundeficiency virus). Da es durch diagnostische Maßnahmen nicht gelingt. hepatitissichere Blutprodukte aus Plasmapools herzustellen, sind verschiedene Verfahren zur Sterilisation von Blutbestandteilen entwickelt worden. Die Pasteurisierung (10 h, 60°C) wird erfolgreich für Albumin verwendet und ist durch die Verwendung von Stabilisatoren, wie Aminosäuren und Mono- bzw. Oligosacchariden und Zuckeralkoholen seit einigen Jahren auch zur Sterilisation empfindlicher Plasmaproteine wie den Gerinnungsfaktoren II, VIII und XIII beschrieben worden (EP 0 018 561). Die Wirksamkeit der Pasteurisation in Gegenwart dieser Stabilisatoren ist noch nicht abzuschätzen und wird zur Zeit überprüft. Die Erhitzung von Immunglobulinen für 10 Minuten auf 63°C führt zu einer drastischen Zunahme ihrer antikomplementären Aktivität, so daß die Pasteurisation zur Sterilisation der Immunglobuline ungeeignet ist (R. van Furth, A. G. P. Braat, P. C. J. Leijh, A. Gardi: Opsonic and physicochemical characteristics of intravenous immunglobulin preparations. Vox Sang. 53: 70 - 75 (1987)).

Die Notwendigkeit, auch Immunglobulinpräparate einer Sterilisationsmaßnahme zu unterziehen, ergibt sich aus einer Reihe von Publikationen aus jüngster Zeit, in denen die Übertragung der Hepatitis B und Non-A/Non-B-Hepatitis durch intravenöse Immunglobulinpräparate beschrieben worden ist:

Björkander, J., Cunningham-Rundles, C., Lundin, P., Söderström, R., Hanson, L. A. (1988): Intravenous immunglobulin prophylaxis causing liver damage in 16 of 77 patients with hypogammaglobulinemia of IgG subclass deficiency. Am. J. Med. 84: 107-111.

John, J. T., Ninan, G. T., Rajagopalan, M. S., John, F. et al (1979): Epidemic hepatitis B caused by commercial human immunglobulin. Lancet I:1074.

Lever, A.M.L., Webster, A.V.D., Brown, D., Thomas H.C. (1984): Non-A, non-B hepatitis occuring in gammaglobulinaemic patients after intravenous immunoglobulin. Lancet II:1062-1064.

Lockner, D., Bratt, G., Lindborg, A., Törnebohm, E. (1987): Acute unidentified hepatitis in a hypogammaglobulinaemic patient on intravenous gammaglobulin successfully treated with interferon. Acta. Med. Scand. 221:413-415.

Williams, P. E., Yap, P. L., Gillon, J. Crawford, R. J., Galea, G., Cuthbertson, B. (1988). Non-A, non-B hepatitis transmission by intravenous immunoglobulin. Lancet II:501.

Die von LoGrippo beschriebene Methode der Kaltsterilisation besteht in der kombinierten Behandlung von Humanplasma mit β-Propiolacton und UV-Bestrahlung (LoGrippo, G. A. u. Hayashi, H.: Henry Ford Hosp. Med. J. 21 (1973), 181; LoGrippo, G. A. und Hartmann, F. W.: Bibl. Haematol. 7 (1958), 225).

Die von LoGrippo publizierten Daten zeigen, daß nur die Kombination von $\beta$-Propiolacton mit der UV-Bestrahlung zu virussicheren Plasmen geführt hat (LoGrippo, G. A.: A ten year clinical study of plasma treated with Betaprone and combined Betaprone plus ultraviolet irradiation. Pacific Medicine and Surgery 72 (1964) 298-302).

In der EP 0 013 901 wird $\beta$-Propiolacton zur Behandlung einer IgM-haltigen Proteinlösung zur Herstellung einer intravenös applizierbaren Immunglobulinlösung verwendet.

Das erfindungsgemäße Verfahren führt bereits ohne $\beta$-Propiolactonbehandlung zu einer mit IgM angereicherten Immunglobulinlösung, deren antikomplemetäre Aktivität ca. 600 CH 50/g Protein beträgt. Durch eine zusätzliche Behandlung mit $\beta$-Propiolacton, vorzugsweise in einer Konzentration von 0,05 bis 0,15 ml pro 100 ml einer 4%igen Immunglobulinlösung, oder Tri-N-Butylphosphat, vorzugsweise in einer Konzentration von 0,10 bis 1,0 ml pro 100 ml einer 5%igen Immunglobulinlösung, kann eine Virusübertragung durch dieses Produkt ausgeschlossen werden. Es genügt also die alleinige Behandlung der Immunglobulinlösung mit $\beta$-Propiolacton ohne zusätzliche UV-Bestrahlung, um auch eine Übertragung der Hepatitis Non-A/Non-B sicher auszuschließen, wie in einem Experiment am Schimpansen gezeigt werden konnte.

Ein weiterer wichtiger Unterschied zwischen dem Verfahren der EP 0 013 901 und dem erfindungsgemäßen Verfahren betrifft die Adsorption mit einem Anionenaustauscher. In der EP 0 013 901 wird die Cohn-Fraktion III noch vor der Octansäure-Behandlung mit dem Anionenaustauscher behandelt, während das erfindungsgemäße Verfahren diesen Verfahrensschritt erst nach der Behandlung der Immunglobulinlösung mit Octansäure und Aktivkohle durchführt, um sicherzustellen, daß während der Aufarbeitung der Cohn-Fraktion III auftretende proteolytische Aktivitäten sicher entfernt werden.

Aufgabe der EP 0 013 901 war es, aus einer IgG-, IgA- und IgM-haltigen Proteinfraktion (Cohn-Fraktion III) eine intravenös applizierbare Lösung dieser Proteine herzustellen. Dafür war die Verwendung von ß-Propiolacton zur Reduzierung der antikomplementären Aktivität in dem Ausgangsmaterial notwendig. Überraschenderweise konnte nun gezeigt werden, daß durch das erfindungsgemäße Verfahren die antikomplementäre Aktivität der Ausgangsfraktion bereits so deutlich verbessert wurde, daß auch andere zur Sterilisation von Blutprodukten geeignete Substanzen, wie z.B. Tri-N-Butylphosphat, anstelle von ß-Propiolacton verwendet werden können. Die Werte der antikomplementären Aktivität (ACA) und der in vivo-Verträglichkeit der nach dem erfindungsgemäßen Verfahren hergestellten Präparate und von Vergleichspräparaten sind in Tab. IV (Anhang) zusammengestellt.

Zur Bestimmung der antikomplemetären Aktivität der Immunglobuline wurde eine definierte Menge des Prüfproduktes mit einer definierten Menge von Meerschweinchen-Komplement inkubiert und die verbleibende Menge Komplement wurde titriert. Die ACA wurde als Verbrauch der CH-50 pro g Immunglobulin angegeben. Die Methode zur Bestimmung der ACA entsprach weitgehend der von M. Mayer publizierten Methode (Mayer, M.M. (1961). Complement and complement fixation. In: Experimental Immunochemistry, 2nd edn., pp 133-240, C. Thomas, Springfield, Il.).

Als Richtwert für intravenös anwendbare IgG-Produkte gilt eine antikomplementäre Aktivität (ACA) von ≤ 1500 CH 50 pro g Protein. Die Cohn-Fraktion III zeigt ACA-Werte von > 1500 CH 50/g Protein und ist erfahrungsgemäß intravenös unverträglich.

Das IgM-haltige Produkt der EP 0 013 901 weist eine ACA von 300 CH 50/g auf und ist erfahrungsgemäß bei der intravenösen Anwendung gut verträglich. Ein in vivo Modell zur Prüfung der i.v. Verträglichkeit ist das Rattenmodell nach Bleeker et al. (W. K. Bleeker, J. Agterberg, G. Righter, A. de Vriesvan Rossen, J. C. Bakker: An animal model for the detection of hypotensive side effects of immunoglobulin preparations. Vox Sang. 52: 281-290 (1987).

Verträglichkeitsparameter für die Immunglobuline in diesem Modell ist der Blutdruck. Intravenös unverträgliche Präparate führen zu einer deutlichen Blutdrucksenkung. Der Vergleich nicht intravenös verträglicher Präparate, dies sind i.m. (intramuskulär) anwendbare Präparate, mit dem Produkt der EP 0 013 901 und dem erfindungsgemäßen Produkt des Beispiels 1 und 2 zeigt, daß die erfindungsgemäß hergestellten Produkte zu einem Blutdruckabfall an der Ratte führen (s. Tab. IV), der deutlich geringer ist als der von i.m. Produkten.

Die folgenden Beispiele erläutern die Erfindung:

Beispiel 1

Pro kg Paste Cohn-Fraktion III wurden 3 kg destilliertes Wasser gegeben. Die Produkttemperatur wurde auf 4°C abgekühlt. Pro 1 kg dest $H_2O$ wurden 0.0055 kg Na-acetat x 3 $H_2O$ zugegeben. Durch Zugabe von 96 %iger Essigsäure wurde der pH-Wert auf pH 5.05 eingestellt. Jeweils 50 kg Paste wurden in 120 l Pufferlösung bei + 4°C suspendiert. Der pH-Wert der Suspension wurde mit 96 %iger Essigsäure auf pH 5.05 eingestellt. Durch Zentrifugation mit einer Cepa-Zentrifuge wurde der Niederschlag abgetrennt. Die vom Niederschlag abgetrennte Lösung wurde auf + 25°C erwärmt. Pro kg Überstand wurden 25 ml Octansäure zugegeben. Die Zugabe der Octansäure erfolgte über Tropftrichter. Der pH-Wert der Lösung wurde auf 4.8 eingestellt und der Ansatz für 1 Stunde bei + 20°C belassen. Pro kg des Ansatzes wurden 4 g Tricalciumphosphat zugegeben und der Ansatz für 45 Minuten bei 20°C belassen, bevor die Suspension zur Entfernung des Niederschlages zentrifugiert wurde. Der Überstand wurde nach der Zentrifugation filtriert und anschließend ultrafiltriert. Die gegen Acetatpuffer ultrafiltrierte Lösung wurde auf einen Proteinwert von 5 % und einen pH-Wert von 6.7 eingestellt. Pro kg dieser Lösung wurden 5 g Aktivkohle unter langsamem Rühren, bei Raumtemperatur gegeben. Die Adsorptionszeit betrug 1 Stunde. Anschließend wurde die Aktivkohle abzentrifugiert und der Überstand filtriert. Zu der filtrierten Lösung wurde in 0.08 M Na-Acetat-Lösung gequollenes DEAE-Sephadex A-50[R] in einer Menge von 40 mg pro g Protein gegeben. Die Sephadex-Adsorption erfolgte bei pH 6.5. Nach Abtrennung des Sephadex wurde der Proteingehalt des Eluats auf 40g/l eingestellt und pro 1 l Überstand 1.40 ml ß-Propiolacton unter pH-Konstanz von pH 8.0 - 8.1 zugegeben. Der pH-Wert wurde durch Zugabe von 1 N NaOH konstant gehalten. Nach der ß-Propiolacton-Behandlung wurde die Lösung sterilfiltriert. Die sterilfiltrierte Lösung wurde zur Einstellung der Ionenkonzentration und des Proteinwertes ultra- und diafiltriert. Diese Lösung wurde sterilfiltriert und in 1-Liter-leersterile Flaschen abgefüllt.

### Beispiel 2

Ausgangsmaterial war, wie in Beispiel 1 beschrieben, die Cohn-Fraktion III, die pro 50 kg Paste in 120 l Pufferlösung, bei + 4°C suspendiert wurde. Der pH-Wert wurde mit 96 %iger Essigsäure auf pH 5.05 eingestellt. Pro kg der Suspension wurden 40 ml Octansäure über einen Tropftrichter bei Raumtemperatur zugegeben. Anschließend wurde für 15 Minuten gerührt und der pH-Wert mit 1 N NaOH auf pH 5.05 eingestellt. Pro kg des Ansatzes wurden anschließend 4 g Tricalciumphosphat zugegeben und der Ansatz für 1 Stunde, unter Rühren, bei Raumtemperatur belassen. Anschließend wurde zentrifugiert. Der Überstand wurde, wie in Beispiel 1 aufgeführt, ultrafiltriert, mit Aktivkohle, DEAD-Sephadex A-50[R]) und $\beta$-Propiolacton behandelt, bevor die Lösung ultrafiltriert, sterilfiltriert und abgefüllt wurde.

### Beispiel 3

Ausgangsmaterial war, wie in Beispiel 1 beschrieben, die Cohn-Fraktion III, die pro 50 kg Paste in 120 l Pufferlösung, bei + 4°C suspendiert wurde. Die Octansäurebehandlung und Calciumphosphat-Adsorption wurde, wie unter Beispiel 1 beschrieben, durchgeführt. Der nach der Zentrifugation filtrierte Überstand wurde mit dem Polyoxyethylenderivat Tween[R] 80 in einer Konzentration von 1% versetzt und 15 Minuten später wurde Tri-N-Butylphosphat bis zu einer Konzentration von 0.3 % zugegeben. Der ansatz wurde für 8 Stunden, bei + 25°C gerührt. Anschließend wurde 5 % Sojabohnenöl dazugegeben, 30 Minuten nach dem Rühren die wässrige Phase von der Ölphase dekantiert und die wässrige Lösung gegen Acetatpuffer ultrafiltriert. Der Proteinwert wurde auf 5 % eingestellt und der pH-Wert auf 6.7. Pro kg dieser Lösung wurden 5 g Aktivkohle unter langsamem Rühren, bei Raumtemperatur gegeben. Die Adsorptionszeit betrug 1 Stunde. Anschließend wurde die Aktivkohle abzentrifugiert und der Überstand filtriert. Zu der filtrierten Lösung wurde in 0.08 M Na-Acetat-Lösung gequollenes DEAE-Sephadex A-50[R] in einer Menge von 40 mg pro g Protein gegeben. Die Sephadex-Adsorption erfolgte bei pH 6.5. Nach Abtrennung des Sephadex wurde die Lösung zur Einstellung der Ionenkonzentration und des Proteinwertes ultra- und diafiltriert. Diese Lösung wurde sterilfiltriert und in 1-Liter leersterile Flaschen abgefüllt.

5

Tab. I

Auswirkung der Aerosilmenge auf die Verteilung der IgG-Subklassen
polyvalenter Immunglobuline

| Produkt | pH | Protein g/1 | IgG g/1 | Gesamtlipide mg/dl | IgG-1 | 2 | 3 | 4 |
|---|---|---|---|---|---|---|---|---|
| | | | | | | Verteilung in % | | |
| RGB-810 | 7.05 | 35.6 | 31.36 | 158 | 69.1 | 21.2 | 5.4 | 4.3 |
| " + 0.5 AE | 6.89 | 34.7 | 30.15 | 28 | 70.8 | 22.1 | 2.7 | 4.4 |
| " + 1.0 AE | 6.78 | 32.4 | 28.72 | 7 | 73.1 | 22.3 | 0.3 | 4.2 |

EP 0 413 187 B1

Tab. II

## Auswirkung der Aerosilmenge auf die Verteilung der IgG-Subklassen in Serumproteinlösungen

| Produkt | pH | Protein | IgG | Gesamtlipide | IgG-1 | 2 | 3 | 4 |
|---|---|---|---|---|---|---|---|---|
| | | | g/l | mg/dl | | | Verteilung in % | |
| RPCS 642 | 7.47 | 52.5 | 6.98 | 351 | 66.9 | 23.0 | 3.1 | 7.0 |
| " + 0.5 % AE | 7.65 | 52.3 | 7.51 | 51 | 65.7 | 22.7 | 3.5 | 8.1 |
| " + 1.0 % AE | 7.64 | 50.9 | 7.51 | 47 | 69.2 | 20.8 | 3.0 | 7.0 |
| " + 2.0 % AE | 7.54 | 48.1 | 6.95 | 41 | 70.7 | 21.8 | 0.4 | 7.1 |

EP 0 413 187 B1

Tab IV

| Antikomplementäre Aktivität (ACA) und in vivo Verträglichkeit an der Ratte | | |
|---|---|---|
| Produkt | ACA/g Protein | Blutdruckänderung in % (n = 6) |
| IgM-Konzentrat nach Beispiel 1 ohne $\beta$-PL | 800 | - 30 |
| IgM-Konzentrat nach Beispiel 1 mit $\beta$-PL | 250 | - 15 |
| IgM-Konzentrat nach Beispiel 2 ohne $\beta$-PL | 600 | - 20 |
| IgM-Konzentrat nach Beispiel 2 mit $\beta$-PL | 180 | - 15 |
| Referenz-IgG-Präparat (handelsübliches i.v. IgG-Präparat) | 80 | - 8 |
| IgM-Konzentrat nach EP 0 013 901 (ohne $\beta$-PL) | >1500 | - 50 |
| IgM-Konzentrat nach EP 0 013 901 (mit $\beta$-PL) | 300 | - 20 |
| Cohn-Fraktion III bzw. i.m. Produkt | >1500 | - 60 |

## Patentansprüche

1.  Verfahren zur Herstellung einer für die intravenöse Applikation geeigneten Immunglobulinlösung durch Behandlung einer durch Fraktionierung aus Hinmanblut erhaltenen Proteinfraktion, die die Immunglobuline des Typs IgG, IgA und IgM in ankonzentrierter Form enthält, dadurch gekennzeichnet, daß
    a) die Proteinfraktion mit Acetatpuffer versetzt,
    b) mit Calciumphosphat und Octansäure behandelt und
    c) zentrifugiert wird,
    d) der Überstand abgetrennt und
    e) mit Aktivkohle behandelt und
    f) einer Adsorption an DEAE-Sephadex A50$^{(R)}$ unterworfen und
    g) die nach Abtrennen des Adsorptionsmittels erhaltene Lösung sterilfiltriert wird.

2.  Verfahren nach Anspruch 1, dadurch gekennzeichnet, daß die im Acetatpuffer unlöslichen Bestandteile durch Zentrifugation entfernt werden.

3.  Verfahren nach einem der Ansprüche 1 oder 2, dadurch gekennzeichnet, daß die erhaltene Immunglobulinlösung mit $\beta$-Propiolacton behandelt wird.

4.  Verfahren nach Anspruch 3, dadurch gekennzeichnet, daß $\beta$-Propiolacton in Mengen von 0,05 bis 0,15 ml pro 100 ml einer 4%igen Immunglobulinlösung verwendet wird.

5.  Verfahren nach einem der Ansprüche 1 oder 2, dadurch gekzeichnet, daß die erhaltene Immunglobulinlösung mit Tri-N-Butylphosphat behandelt wird.

6.  Verfahren nach Anspruch 5, dadurch gekennzeichnet, daß Tri-N-Butylphosphat in Mengen von 0,10 bis 1,0 ml pro 100 ml einer 5%igen Immunglobulinlösung verwendet wird.

## Claims

1.  A process for the preparation of an immunoglobulin solution suitable for intravenous administration by the treatment of a protein fraction obtained from human blood by fractionation and containing IgG-, IgA- and IgM-type immunoglobulins in concentrated form, characterized in that
    a) acetate buffer is added to the protein fraction,
    b) the resulting mixture is treated with calcium phosphate and octanoic acid and
    c) centrifuged,
    d) the supernatant is separated off,
    e) treated with activated charcoal and
    f) subjected to adsorption on DEAE-Sephadex A50$^{(R)}$, and
    g) the solution obtained after separation of the adsorbent is filtered under sterile conditions.

**2.** A process according to Claim 1, characterized in that the constituents insoluble in the acetate buffer are removed by centrifugation.

**3.** A process according to one of Claims 1 or 2, characterized in that the immunoglobulin solution obtained is treated with $\beta$-propiolactone.

**4.** A process according to Claim 3, characterized in that $\beta$-propiolactone is used in amounts of 0.05 to 0.15 ml per 100 ml of a 4% immunoglobulin solution.

**5.** A process according to one of Claims 1 or 2, characterized in that the immunoglobulin solution obtained is treated with tri-n-butyl phosphate.

**6.** A process according to Claim 5, characterized in that tri-n-butyl phosphate is used in amounts of 0.10 to 1.0 ml per 100 ml of a 5% immunoglobulin solution.

**Revendications**

**1.** Procédé de préparation d'une solution d'immunoglobulines convenant à l'administration intraveineuse par traitement d'une fraction protéique obtenue par fractionnement de sang humain et qui contient les immunoglobulines du type IgG, IgA et IgM sous forme concentrée, caractérisé en ce que :
a) on ajoute un tampon acétate à la fraction protéique,
b) on la traite à l'aide de phosphate calcique et d'acide caprylique et
c) on la centrifuge,
d) on sépare le surnageant et
e) on le traite à l'aide de charbon actif et
f) on le soumet à une adsorption sur DEAE-Sephadex A 50[(R)] et
g) on soumet la solution, obtenue après séparation de l'adsorbant, à une filtration stérile

**2.** Procédé selon la revendication 1, caractérisé en ce que les composants insolubles dans le tampon acétate sont séparés par centrifugation.

**3.** Procédé selon l'une des revendications 1 et 2, caractérisé en ce que la solution d'immunoglobulines obtenue est traitée par de la $\beta$-propiolactone.

**4.** Procédé selon la revendication 3, caractérisé en ce qu'on utilise la $\beta$-propiolactone en quantités de 0,05 à 0,15 ml par 100 ml d'une solution d'immunoglobulines à 4 %.

**5.** Procédé selon une des revendications 1 et 2, caractérisé en ce qu'on traite la solution d'immunoglobulines obtenue à l'aide de phosphate de tri-n-butyle.

**6.** Procédé selon la revendication 5, caractérisé en ce qu'on utilise le phosphate de tri-n-butyle en quantités de 0,10 à 1,0 ml par 100 ml d'une solution d'immunoglobulines à 5 %.

9

**Abb. I**

### Einstufenverfahren

---

┌─────────────────────────┐
│ **Cohn Fraktion III** │
└─────────────────────────┘

┌────────────────────────────────────────────────┐
│ 0.005 M Acetatpuffer; Protein: ~ 4 - 5 % │
│ + 40 ml Octansäure auf 1 l │
│ + 4 g $Ca_3$ $(PO_4)_2$ auf 1 l │
└────────────────────────────────────────────────┘

Zentrifugation

┌─────────────────────────────────┐
│ Ultrafiltration; 5 % Protein │
└─────────────────────────────────┘

┌─────────────────────────┐
│ Aktivkohle: 5 g/l │
└─────────────────────────┘

┌─────────────────────────┐
│ DEAE-Sephadex A-50 │
│ 40 mg/g Protein │
└─────────────────────────┘

┌────────────────────────────────────────────────┐
│ β-Propiolacton: 1.25 ± 0.25 ml auf 1 l │    bei 4 % Protein
│ 0.12 ml auf 100 ml │
└────────────────────────────────────────────────┘

┌─────────────────────────┐
│ Sterilfiltration │
└─────────────────────────┘

── UF

┌─────────────────────────┐
│ Lösung von IgG, IgA, IgM │
└─────────────────────────┘

Abb. II

**Zweistufenverfahren**

```
              ┌─────────────────────────┐
              │     Cohn Fraktion III    │
              └─────────────────────────┘
                           │
     ┌────────────────────────────────────────────┐
     │ 0.05 M Acetatpuffer; Protein: ~ 4-5 %       │
     └────────────────────────────────────────────┘
                           │
                                    Zentrifugation

          ┌──────────────────────────────────┐
          │     25 ml Octansäure auf 1 l      │
          │   4 g Ca₃ (PO₄)₂ auf 1 l          │
          └──────────────────────────────────┘
                           │
                                    Zentrifugation

          ┌──────────────────────────────────┐
          │   Ultrafiltration; 5 % Protein    │
          └──────────────────────────────────┘
                           │
               ┌─────────────────────┐
               │  Aktivkohle : 5 g/l │
               └─────────────────────┘
                           │
             ┌──────────────────────────┐
             │    DEAE-Sephadex A-50     │
             │      40 mg/g Protein      │
             └──────────────────────────┘
                           │
    ┌──────────────────────────────────────────┐
    │ β-Propiolacton: 1.25 ± 0.25 ml auf 1 l    │     bei 4 % Protein
    │         0.12 ml auf 100 ml                │
    └──────────────────────────────────────────┘
                           │
             ┌─────────────────────────┐
             │     Sterilfiltration     │
             └─────────────────────────┘
                           │
                           ├──── UF, Sterilfiltr.
                           │
          ┌──────────────────────────────────┐
          │   Lösung von IgG, IgA, IgM        │
          └──────────────────────────────────┘
```

Cohn Fraktion III

0.05 M Acetatpuffer; Protein: ~ 4-5 %

Zentrifugation

25 ml Octansäure auf 1 l

4 g $Ca_3$ $(PO_4)_2$ auf 1 l

Zentrifugation

Ultrafiltration; 5 % Protein

Aktivkohle : 5 g/l

DEAE-Sephadex A-50
40 mg/g Protein

β-Propiolacton: 1.25 ± 0.25 ml auf 1 l
0.12 ml auf 100 ml        bei 4 % Protein

Sterilfiltration

UF, Sterilfiltr.

Lösung von IgG, IgA, IgM